(19) Europäisches Patentamt
European Patent Office
Office européen des brevets

(11) EP 0 815 843 A1

(12) DEMANDE DE BREVET EUROPEEN

(43) Date de publication:
**07.01.1998 Bulletin 1998/02**

(51) Int Cl.6: **A61K 7/48**, A61K 7/00, A61K 7/02, A61K 7/06

(21) Numéro de dépôt: **97401205.6**

(22) Date de dépôt: **30.05.1997**

(84) Etats contractants désignés:
**DE ES FR GB IT**

(30) Priorité: **28.06.1996 FR 9608112**

(71) Demandeur: **L'OREAL**
**75008 Paris (FR)**

(72) Inventeurs:
- **Terren, Nadia**
**94550 Chevilly Larue (FR)**
- **Favre, Sophie**
**94550 Chevilly Larue (FR)**

(74) Mandataire: **Dodin, Catherine**
**L'Oreal-D.P.I.,**
**90, rue du Général Roguet**
**92583 Clichy Cédex (FR)**

# (54) Composition comprenant un polymère poly(acide 2-acrylamido 2-métylpropane sulfonique) réticulé

(57) L'invention concerne notamment une composition cosmétique, dermatologique, hygiénique et/ou pharmaceutique, se présentant sous la forme de, et/ou comprenant, un gel aqueux, une solution aqueuse, hydroalcoolique ou multiphasée, comprenant en outre au moins un polymère poly(acide 2-acrylamido 2-méthylpropane sulfonique) réticulé et neutralisé à au moins 90%, et des colorants hydrosolubles.

L'invention concerne également une composition cosmétique, dermatologique, hygiénique et/ou pharmaceutique, se présentant sous la forme de, et/ou comprenant, une émulsion huile-dans-eau, une émulsion eau-dans-huile, une émulsion multiple ou une solution multiphasée, comprenant en outre au moins un polymère poly(acide 2-acrylamido 2-méthylpropane sulfonique) réticulé et neutralisé à au moins 90%, et des pigments.

EP 0 815 843 A1

## Description

La présente invention a pour objet une composition notamment cosmétique pouvant en particulier se présenter sous forme d'une émulsion, et susceptible d'être utilisée pour le soin et/ou le maquillage de la peau, des semi-muqueuses, des muqueuses et/ou des phanères.

Les compositions cosmétiques de maquillage comprennent généralement des corps gras tels que des huiles et des cires, et une phase particulaire généralement composée de charges et de pigments. Elles peuvent ainsi se présenter, par exemple dans les cas des rouges à lèvres, sous forme de stick ou bâton ou sous forme de pâte souple. Les compositions de maquillage peuvent également comprendre de l'eau ou une phase hydrophile, et se présenter alors notamment sous forme d'émulsion huile-dans-eau, eau-dans-huile, émulsion multiple, solution ou gel aqueux, notamment lorsqu'il s'agit d'un fond de teint, de crème teintée, de crème de soin ou d'un produit solaire.
On a constaté que lorsque ces diverses compositions cosmétiques sont appliquées sur la peau, les muqueuses ou les semi-muqueuses, elles présentent l'inconvénient de transférer. On entend par là que la composition est susceptible de se déposer, au moins en partie, sur certains supports avec lesquels elle est mise en contact, tels que, par exemple, un verre, un vêtement ou la peau. En se déposant, ladite composition laisse une trace sur ledit support. Il s'en suit donc une persistance médiocre de la composition sur la peau ou les muqueuses, d'où la nécessité de renouveler régulièrement son application.
Un autre inconvénient de ces compositions réside dans le problème de migration. On a en effet constaté que certaines compositions avaient tendance à se propager à l'intérieur des ridules et/ou des rides de la peau, dans le cas des fonds de teint; dans les ridules qui entourent les lèvres, dans le cas des rouges à lèvres; dans les plis de la paupière, dans le cas des fards à paupières. On a également constaté, dans le cas notamment des fards à paupières, l'apparition de stries dans le maquillage, générées par les mouvements des paupières. Tous ces phénomènes engendrent un effet inesthétique, que la consommatrice souhaite bien évidemment éviter.

La présente invention a pour but de proposer une composition qui permet d'obtenir un film de très bonne tenue, qui ne transfère pas et ne tache pas un support avec lequel il serait en contact.

L'invention a donc pour premier objet l'utilisation d'un polymère poly(acide 2-acrylamido 2-méthylpropane sulfonique) réticulé et neutralisé à au moins 90% dans une composition cosmétique, dermatologique, hygiénique et/ou pharmaceutique, afin de limiter, diminuer et/ou de supprimer le transfert et/ou la migration de ladite composition; et/ou afin d'améliorer la tenue de ladite composition.
L'invention a également pour objet l'utilisation d'un tel polymère comme agent permettant de limiter, diminuer et/ou de supprimer le transfert et/ou la migration de ladite composition, et/ou comme agent permettant d'améliorer la tenue de ladite composition.
Un autre objet de l'invention est une composition cosmétique, dermatologique, hygiénique et/ou pharmaceutique, se présentant sous la forme de, et/ou comprenant, une émulsion huile-dans-eau, une émulsion eau-dans-huile, une émulsion multiple ou une solution multiphasée, comprenant en outre au moins un polymère poly(acide 2-acrylamido 2-méthylpropane sulfonique) réticulé et neutralisé à au moins 90%, et des pigments.
Encore un objet de l'invention est une composition cosmétique, dermatologique, hygiénique et/ou pharmaceutique, se présentant sous la forme de, et/ou comprenant, un gel aqueux, une solution aqueuse, hydroalcoolique ou multiphasée, comprenant en outre au moins un polymère poly(acide 2-acrylamido 2-méthylpropane sulfonique) réticulé et neutralisé à au moins 90%, et des colorants hydrosolubles.
Un autre objet de l'invention est une composition de maquillage ou de soin sans transfert, comprenant au moins un tel polymère.
L'invention a encore pour objet un procédé pour limiter, diminuer et/ou empêcher le transfert d'une composition cosmétique, dermatologique, hygiénique et/ou pharmaceutique, notamment une composition de maquillage ou de soin de la peau, des muqueuses, des semi-muqueuses et/ou des phanères, consistant à introduire dans ladite composition au moins un polymère poly(acide 2-acrylamido 2-méthylpropane sulfonique) réticulé et neutralisé à au moins 90%.

On a de plus constaté que les compositions selon l'invention ne migrent pas au cours du temps.
D'autre part, les compositions telles que les produits pour le soin, l'hygiène ou le maquillage qui contiennent le polymère selon l'invention, s'étalent de façon homogène sur la surface locale à traiter; les compositions capillaires s'étalent et se répartissent de façon régulière le long des fibres kératiniques et ne ruissellent pas sur le front, la nuque ou le visage ou dans les yeux. Ceci est notamment dû au polymère utilisé, qui, grâce à ses propriétés particulières d'agent épaississant et/ou gélifiant, permet d'obtenir un très grand nombre de formulations cosmétiques contenant des supports de nature différente.
De plus, les compositions selon l'invention supportent les actifs cosmétiques classiques, et spécifiques, notamment les hydroxyacides.
Enfin, leurs propriétés cosmétiques sont très intéressantes : elles procurent une certaine fraîcheur à l'application, ainsi qu'une grande douceur.

Les compositions selon l'invention comprennent donc au moins un polymère poly(acide 2-acrylamido 2-méthylpropane sulfonique) réticulé et neutralisé à au moins 90%. Ce polymère poly(acide 2-acrylamido 2-méthylpropane sulfonique) réticulé et pratiquement ou totalement neutralisé est généralement hydrosoluble ou gonflable dans l'eau.

Ces polymères sont en général caractérisés par le fait qu'ils comprennent, distribués de façon aléatoire :

a) de 90 à 99,9% en poids de motifs de formule générale (1) suivante :

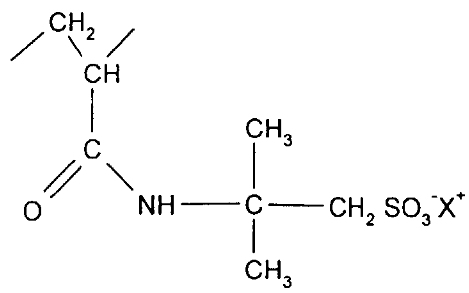

(1)

dans laquelle $X^+$ désigne un cation ou un mélange de cations, au plus 10% molaire des cations $X^+$ pouvant être des protons $H^+$ ; et

b) de 0,01 à 10% en poids de motifs réticulants provenant d'au moins un monomère ayant au moins deux doubles liaisons oléfiniques ; les proportions en poids étant définies par rapport au poids total du polymère.

De façon préférentielle, les polymères de l'invention comportent un nombre de motifs de formule (1) dans une quantité suffisamment élevée pour obtenir des particules de polymère dont le volume hydrodynamique en solution d'eau présente un rayon allant de 10 à 500 nm et dont la distribution est homogène et unimodale.

Le volume hydrodynamique est déterminé par le coefficient de diffusion D selon STOKES-EINSTEIN selon la méthode de caractérisation d'un mélange de polymères par diffusion au LASER décrite dans l'article de CHI WU et al, Macromolecules, 1995, 28,4914-4919.

Les polymères selon l'invention plus particulièrement préférés comprennent de 98 à 99,5 % en poids de motifs de formule (1) et de 0,2 à 2 % en poids de motifs réticulants.

Le cation $X^+$ représente un cation ou un mélange de cations choisis en particulier parmi un proton, un cation de métal alcalin, un cation équivalent de celui d'un métal alcalino-terreux ou l'ion ammonium. Le cation $X^+$ préférentiel est le cation $NH_4^+$.

Plus particulièrement, 90 à 100% mole des cations sont des cations $NH_4^+$ et 0 à 10% mole sont des protons ($H^+$).

Les monomères de réticulation ayant au moins deux doubles liaisons oléfiniques sont choisis par exemple parmi le dipropylèneglycol-diallyléther, les polyglycoldiallyléthers, le triéthylèneglycol-divinyléther, l'hydroquinone-diallyléther, le tétrallyl-oxéthanoyle ou d'autres allyl ou vinyléthers alcools polyfonctionnels, le diacrylate de tétraéthylèneglycol, la triallylamine, le triméthylolpropane-diallyléther, le méthylen-bis-acrylamide ou le divinylbenzène.

Les monomères de réticulation ayant au moins deux doubles liaisons oléfiniques sont plus particulièrement choisis parmi ceux répondant à la formule générale (2) suivante :

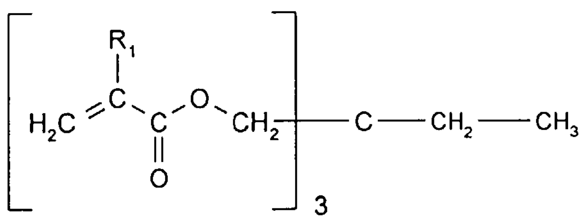

(2)

dans laquelle $R_1$ désigne un atome d'hydrogène ou un radical alkyle en $C_1$-$C_4$ et plus particulièrement le radical méthyle (triméthylol propane triacrylate).

La réaction de polymérisation des polymères de l'invention produit non seulement des chaînes linéaires mais aussi des molécules de polymère ramifiées ou réticulées. Ces molécules peuvent être caractérisées notamment par leur comportement rhéologique dans l'eau mais plus particulièrement par la diffusion de la lumière dynamique.

Dans le cas de la caractérisation des molécules par la diffusion de la lumière dynamique, on mesure la distribution du volume hydrodynamique des structures du polymère. Les macromolécules dissoutes dans l'eau sont flexibles et entourées par une enveloppe de solvatation formée de molécules d'eau. Avec des polymères chargés comme ceux

de l'invention, la taille des molécules dépend de la quantité de sel dans l'eau. Dans les solvants polaires, la charge uniforme le long de la chaîne principale du polymère conduit à une expansion importante de la chaîne polymérique. Le fait d'accroître la quantité de sel augmente la quantité d'électrolyte dans le solvant et fait écran aux charges uniformes du polymère. En plus des molécules transportées dans l'enveloppe de solvatation, les molécules de solvant sont fixées dans les cavités du polymère. Dans ce cas, les molécules de solvant font partie des macromolécules en solution et se déplacent à la même vitesse moyenne. Ainsi, le volume hydrodynamique décrit la dimension linéaire de la macromolécule et de ces molécules de solvatation.

Le volume hydrodynamique $v_h$ est déterminé par la formule suivante :

$$v_h = \frac{M \times (V_2 + dV_1)}{N_A}$$

avec:

- $N_A$ désignant le nombre d'Avogadro ;
- M est la masse en gramme de la macromolécule non dissoute,
- $V_1$ désignant le volume spécifique du solvant ;
- $V_2$ désignant le volume spécifique de la macromolécule ;
- d la masse en grammes du solvant qui est associé avec 1 gramme de macromolécule non dissoute.

Si la particule hydrodynamique est sphérique, il est alors facile de calculer à partir du volume hydrodynamique le rayon hydrodynamique par la formule :

$$v_h = \frac{4\Pi R^3}{3}$$

avec R désignant le rayon dynamique.

Les cas où les particules hydrodynamiques sont des sphères parfaites sont extrêmement rares. La majorité des polymères synthétiques impliquent des structures compactées ou des ellipsoïdes à haute excentricité. Dans ce cas, la détermination du rayon s'effectue sur une sphère qui est équivalente d'un point de vue frottement à la forme de la particule considérée.

En règle générale, on travaille sur des distributions de poids moléculaire et donc sur des distributions de rayon et de volume hydrodynamique. Pour les systèmes polydispersés, on doit calculer la distribution des coefficients de diffusion. De cette distribution , on en déduit les résultats relatifs à la distribution radiale et à la distribution des volumes hydrodynamiques.

Les volumes hydrodynamiques des polymères de l'invention sont en particulier déterminés par diffusion de la lumière dynamique à partir des coefficients de diffusion D selon STOKES-EINSTEIN de formule : $D = kT/6\Pi\eta R$ où k est la constante de Boltzmann, T la température absolue en degrés Kelvin, $\eta$ est la viscosité du solvant (eau) et R est le rayon hydrodynamique.

Ces coefficients de diffusion D sont mesurés selon la méthode de caractérisation d'un mélange de polymères par diffusion au LASER décrite dans les références suivantes :

(1) Pecora, R ; Dynamic Light Scattering ; Plenium Press, New York, 1976 ;
(2) Chu, B ; Dynamic Light Scattering ; Academic Press, New York, 1994 ;
(3) Schmitz, KS ; Introduction to Dynamic Light Scattering ; Academic Press, New York, 1990 ;
(4) Provincher S.W. ; Comp. Phys., 27, 213, 1982 ;
(5) Provincher S.W. ; Comp. Phys., 27, 229, 1982 ;
(6) ALV Laservertriebgesellschaft mbH, Robert Bosch Str. 47, D-63225 Langen, Germany;
(7) ELS-Reinheimer Strasse 11, D-64846 Gross-Zimmern, Germany ;
(8) CHI WU et al, Macromolecules, 1995, 28,4914-4919.

Les polymères particulièrement préférés sont ceux présentant une viscosité mesurée au viscosimètre BROOKFIELD, mobile 4, à une vitesse de rotation de 100 tours/minutes, à 25°C, et en solution aqueuse à 2 % en poids, supérieure ou égale à 1000 cps et plus préférentiellement allant de 5000 cps à 40000 cps et plus particulièrement de 6500 cps à 35000 cps.

Les poly(acide 2-acrylamido 2-méthylpropane sulfonique) réticulés selon l'invention peuvent être obtenus selon

le procédé de préparation comprenant les étapes suivantes :

(a) on disperse ou on dissout le monomère acide 2-acrylamido 2-méthylpropane sulfonique sous forme libre dans une solution de tertio-butanol ou d'eau et de tertio-butanol;
(b) on neutralise la solution ou la dispersion de monomère obtenue en (a) par une ou plusieurs bases minérales ou organiques, de préférence l'ammoniaque $NH_3$, dans une quantité permettant d'obtenir un taux de neutralisation des fonctions acides sulfoniques du polymère allant de 90 à 100%;
(c) on ajoute à la solution ou dispersion obtenue en (b), le ou les monomères réticulants;
(d) on effectue une polymérisation radicalaire classique en présence d'amorceurs de radicaux libres à une température allant de 10 à 150°C; le polymère précipitant dans la solution ou la dispersion à base de tertio-butanol.

Les poly(acide 2-acrylamido 2-méthylpropane sulfonique) réticulés, pratiquement ou totalement neutralisés peuvent être présents dans les compositions selon l'invention à une concentration de 0,01 à 20% en poids par rapport au poids total de la composition, de préférence 0,1 à 5% en poids, et plus préférentiellement 0,4 à 2% en poids.

Les compositions de l'invention contiennent en outre un milieu cosmétiquement, hygiéniquement, pharmaceutiquement ou dermatologiquement acceptable, c'est-à-dire un milieu compatible avec toutes les matières kératiniques telles que la peau, les ongles, les cheveux, les cils et sourcils, les muqueuses et les semi-muqueuses, et toute autre zone cutanée du corps et du visage.

Lesdites compositions contiennent un milieu aqueux cosmétiquement et/ou dermatologiquement acceptable. Dans une forme préférée de réalisation, la composition selon l'invention se présente sous la forme d'une émulsion huile-dans-eau. Toutefois, elle peut également se présenter sous la forme d'une émulsion eau-dans-huile, d'une émulsion multiple, d'un gel aqueux, ou d'une solution aqueuse, hydroalcoolique ou multiphasée, notamment eau/poudre et eau/huile/poudres.

La phase aqueuse de l'émulsion selon l'invention peut comprendre de l'eau, une eau florale telle que l'eau de bleuet et/ou une eau minérale telle que l'eau de VITTEL, l'eau de LUCAS ou l'eau de LA ROCHE POSAY.

Ladite phase aqueuse peut être présente à une teneur de 15 à 99,5 % en poids par rapport au poids total de la composition, de préférence 40 à 80% en poids lorsque la composition se présente sous forme d'émulsion huile-dans-eau, ou de préférence 85 à 95% en poids lorsque la composition se présente sous la forme d'un gel ou d'une solution aqueuse.

En outre, la phase aqueuse peut comprendre de 0 % à 14 % en poids, par rapport au poids total de la phase aqueuse, d'un monoalcool inférieur en $C_2$-$C_6$ et/ou d'un polyol tel que le glycérol, le butylèneglycol, l'isoprène glycol, le propylèneglycol, le polyéthylèneglycol.

Les compositions selon l'invention peuvent comprendre une phase grasse, notamment constituée de corps gras liquides à 25°C, tels que des huiles d'origine animale, végétale, minérale ou synthétique.

Lorsque la composition selon l'invention se présente sous la forme d'une émulsion, ladite phase grasse peut être comprendre toute huile cosmétiquement acceptable, dans la mesure où ladite huile permet, en mélange avec la phase aqueuse et les éventuels additifs, l'obtention d'une émulsion stable, c'est-à-dire d'une émulsion qui ne casse pas, qui reste sous forme d'une phase unique pendant au moins 24 heures après stockage à 25°C, sans phénomène de crémage ou de relarguage d'huile.

Les huiles susceptibles d'être employées peuvent éventuellement être volatiles. On entend par huile volatile, tout composé susceptible de s'évaporer au contact de la peau. De préférence, on utilise des huiles dont le point éclair est suffisamment élevé pour permettre l'utilisation de ces huiles en formulation, et suffisamment bas pour obtenir l'effet évanescent souhaité. On emploie de préférence des huiles dont le point éclair est de l'ordre de 40-100°C.

On peut ainsi citer les huiles siliconées volatiles, telles que :

- les silicones volatiles cycliques ayant de 3 à 8 atomes de silicium et de préférence de 4 à 6. Il s'agit par exemple de la cyclotétradiméthylsiloxane, de la cyclopentadiméthylsiloxane ou de la cyclohexadiméthylsiloxane,
- les cyclocopolymères du type diméthylsiloxane/méthylalkylsiloxane, tels que la SILICONE FZ 3109 vendue par la société UNION CARBIDE , qui est un cyclocopolymère diméthylsiloxane/méthyloctylsiloxane,
- les silicones volatiles linéaires ayant de 2 à 9 atomes de silicium. Il s'agit par exemple de l'hexaméthyldisiloxane, de l'hexyl heptaméthyltrisiloxane ou de l'octyl heptaméthyltrisiloxane.

On peut également citer les huiles volatiles hydrocarbonées, telles que les isoparaffines et notamment l'isododécane.

Parmi les huiles non volatiles, on peut citer :

- les polyalkyl($C_1$-$C_{20}$) siloxanes et notamment ceux à groupements terminaux triméthylsilyle, de préférence ceux dont la viscosité est inférieure à 0,06 $m^2/s$ parmi lesquels on peut citer les polydiméthylsiloxanes linéaires et les alkylméthylpolysiloxanes tels que la cétyldiméthicone (nom CTFA),

- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées, notamment celles de formule :

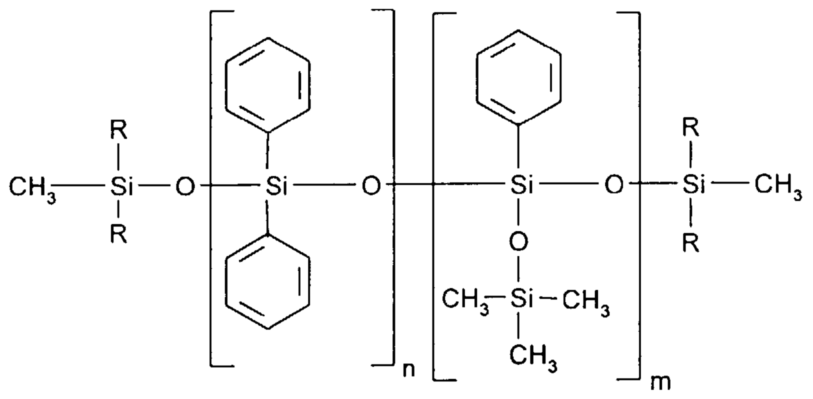

(I)

dans laquelle R est un radical alkyle en C1-C30, un radical aryle ou un radical aralkyle, n est un nombre entier compris entre 0 et 100, et m est un nombre entier compris entre 0 et 100, sous réserve que la somme est comprise entre 1 et 100.

- les huiles d'origine animale, végétale ou minérale, telles que l'huile de paraffine, de vaseline, le perhydrosqualène, l'huile d'abricot, l'huile de germes de blé, d'amande douce, de calophyllum, de sésame, de macadamia, de pépins de raisin, de colza, de coprah, d'arachide, de palme, de ricin, d'avocat, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides, les huiles fluorées et perfluorées.

Dans un mode de réalisation particulier de l'invention, on peut préparer une émulsion ne comprenant que des corps gras siliconés, tels que des huiles cycliques volatiles éventuellement en mélange avec des PDMS et/ou des huiles siliconées phénylées, ou encore tels que des gommes de silicone, notamment phénylées et/ou hydroxylées, en mélange avec des huiles siliconées éventuellement volatiles.

Lorsque la composition se présente sous forme d'une émulsion huile-dans-eau, la phase grasse de l'émulsion peut être présente à une teneur de 2 % à 40 % en poids par rapport au poids total de l'émulsion, de préférence de 3 % à 30 % en poids et en particulier de 3 % à 20 % en poids.

La composition selon l'invention peut comprendre en outre d'autres corps gras, qui peuvent être choisis par l'homme du métier sur base de ses connaissances générales, de manière à conférer à la composition finale les propriétés souhaitées, par exemple en consistance, en texture et/ou en transfert. Ces corps gras additionnels peuvent être des cires, des gommes et/ou des corps gras pâteux d'origine animale, végétale, minérale ou synthétique, ainsi que leurs mélanges.

On peut notamment citer :

- les gommes de silicones,
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; des huiles hydrogénées concrètes à 25°C; des lanolines; des esters gras concrets à 25°C; les cires de silicone; les cires fluorées.

La composition selon l'invention peut en outre comprendre un ou plusieurs solvants organiques cosmétiquement acceptables (tolérance, toxicologie et toucher acceptables). Ces solvants organiques peuvent représenter de 0 % à 98 % du poids total de la composition. Ils peuvent être choisis dans le groupe constitué par les solvants organiques hydrophiles, les solvants organiques lipophiles, les solvants amphiphiles ou leurs mélanges.

Parmi les solvants organiques hydrophiles, on peut citer par exemple des monoalcools inférieurs linéaires ou ramifiés ayant de 1 à 8 atomes de carbone comme l'éthanol, le propanol, le butanol, l'isopropanol, l'isobutanol ; des polyéthylène glycols ayant de 6 à 80 oxydes d'éthylène ; des polyols tels que le propylène glycol, l'isoprène glycol, le butylène glycol, le glycérol, le sorbitol ; les mono- ou di-alkyle d'isosorbide dont les groupements alkyle ont de 1 à 5 atomes de carbone ;

les éthers de glycol comme le diéthylène glycol mono-méthyl ou mono-éthyl éther et les éthers de propylène glycol comme le dipropylène glycol méthyl éther.
Comme solvants organiques amphiphiles, on peut citer des polyols tels des dérivés de polypropylène glycol (PPG) tels que les esters de polypropylène glycol et d'acide gras, de PPG et d'alcool gras comme le PPG-23 oléyl éther et le PPG-36 oléate.
Comme solvants organiques lipophiles, on peut citer par exemple les esters gras tels que l'adipate de diisopropyle, l'adipate de dioctyle, les benzoates d'alkyle.

Lorsque la composition selon l'invention se présente sous la forme d'une émulsion, elle peut éventuellement comprendre en outre un tensioactif, bien que cela ne soit pas nécessaire pour l'obtention d'une émulsion stable et fine. Elle peut également comprendre 0 à 5 % en poids, par rapport au poids total de l'émulsion, d'au moins un co-émulsionnant qui peut être choisi parmi le monostéarate de sorbitan oxyéthyléné, des alcools gras tels que l'alcool stéarylique ou l'alcool cétylique, ou des esters d'acides gras et de polyols tels que le stéarate de glycéryle.

En outre, l'émulsion selon l'invention peut comprendre un ou plusieurs agents épaississants dans des concentrations préférentielles allant de 0 à 6 % en poids, par rapport au poids total de l'émulsion.
L'agent épaississant peut être choisi parmi :

- les biopolymères polysaccharidiques comme la gomme de xanthane, la gomme de caroube, la gomme de guar, les alginates, les celluloses modifiées telles que l'hydroxyéthylcellulose, la méthylcellulose, l'hydroxypropylcellulose et la carboxyméthylcellulose,
- les polymères synthétiques comme les acides polyacryliques tels que les polymères poly(méth)acrylates de glycéryl tels que l'HISPAGEL ou le LUBRAGEL des sociétés HISPANO QUIMICA ou GARDIAN, la polyvinylpyrrolidone, l'alcool polyvinylique, les polymères réticulés d'acrylamide et d'acrylate d'ammonium tels que le PAS 5161 ou BOZEPOL C de HOECHST; les copolymères acrylate/octylacrylamide tels que le Dermacryl de National Starch; les polymères à base de polyacrylamide tels que le SEPIGEL 305 de SEPPIC, les polymères réticulés d'acrylamide et de chlorure de méthacryloyloxyéthyltriméthylammonium tels que le SALCARE SC 92 de ALLIED COLLOIDS,
- le silicate de magnésium et d'aluminium.

De plus, on a constaté que, de manière étonnante, les polymères selon l'invention permettent de préparer des gels aqueux et des émulsions dans une large gamme de pH, notamment de pH acide inférieur à 7, tout en leur conservant une viscosité stable dans le temps à température ambiante ou à des températures plus élevées. Ainsi, la composition selon l'invention peut comprendre au moins un composé acide, c'est-à-dire au moins un composé dont une solution ou une dispersion aqueuse présente un pH inférieur ou égal à 7.
Parmi ces composés acides, on peut citer notamment les hydroxyacides et les $\alpha$-et $\beta$-cétoacides.
Les hydroxyacides utilisés conformément à la présente invention sont choisis préférentiellement parmi les $\alpha$-hydroxyacides, l'acide $\eta$-octanoyl-5-salicylique et/ou l'acide salicylique.
Les $\alpha$-hydroxyacides auxquels s'applique l'invention peuvent être linéaires, ramifiés ou cycliques, saturés ou insaturés. Les atomes d'hydrogène de la chaîne carbonée peuvent, en outre, être substitués par des halogènes, des radicaux halogénés, alkylés, acylés, acyloxylés, alcoxy carbonylés ou alcoxylés ayant de 2 à 28 atomes de carbone. L'$\alpha$-hydroxyacide utilisable selon l'invention peut consister en un acide mono- ou polycarboxylique comportant une ou plusieurs fonctions hydroxy, l'une au moins de ces fonctions hydroxy devant occuper une position a sur ledit acide. De préférence, les $\alpha$-hydroxyacides de l'invention sont des $\alpha$-hydroxyalcanoïques ayant de 2 à 18 atomes de carbone.
Comme $\alpha$-hydroxyacides utilisables dans l'invention, on peut citer notamment les acides glycolique, lactique, malique, tartrique, citrique, mandélique, $\alpha$-hydroxycaprylique, $\alpha$-hydroxyhexanoïque, $\alpha$-hydroxydécanoïque, $\alpha$-hydroxydodécanoïque, $\alpha$-hydroxytétradécanoïque, $\alpha$-hydroxyhexadécanoïque, $\alpha$-hydroxyoctadécanoïque, $\alpha$-hydroxyeisanoïque, $\alpha$-hydroxydocosanoïque, $\alpha$-hydroxyhexacosanoïque et $\alpha$-hydroxyoctacosanoïque.
On peut encore citer l'acide ascorbique, l'acide kojique, l'acide caféique, l'acide salicylique et ses dérivés, ainsi que tous les composés naturels ou synthétiques contenant de tels acides, comme les extraits végétaux et plus spécialement les extraits de fruits.
Les composés acides peuvent être présents dans les compositions de l'invention à des teneurs de 0 à 10 % en poids, par rapport au poids total de la composition, de préférence de 0,2 % à 5 % en poids.

La composition selon l'invention peut comprendre une phase particulaire qui peut comprendre des pigments et/ou des nacres et/ou des charges habituellement utilisés dans les compositions cosmétiques.
Par pigments, il faut comprendre des particules blanches ou colorées, minérales ou organiques, insolubles dans le milieu, destinées à colorer et/ou opacifier la composition. Par charges, il faut comprendre des particules incolores ou blanches, minérales ou de synthèse, lamellaires ou non lamellaires, destinées à donner de la douceur, de la matité et de l'uniformité au maquillage. Par nacres, il faut comprendre des particules irisées qui réfléchissent la lumière.

Les pigments peuvent être présents à raison de 0-20 % en poids, par rapport au poids total de la composition, et de préférence à raison de 2-15 %. Ils peuvent être blancs ou colorés, minéraux et/ou organiques, de taille usuelle ou

nanométrique. On peut citer, parmi les pigments et les nanopigments minéraux, les dioxydes de titane, de zirconium ou de cérium, ainsi que les oxydes de zinc, de fer ou de chrome, les nanotitanes, le bleu ferrique. Parmi les pigments organiques, on peut citer le noir de carbone, et les laques de baryum, strontium, calcium, aluminium.

Lorsque la composition se présente sous forme d'une émulsion huile-dans-eau, les pigments sont de préférence hydrophobes ou rendus hydrophobes, c'est-à-dire qu'ils peuvent être traités de manière à rendre leur surface hydrophobe; ce traitement peut être effectué selon les méthodes connues de l'homme du métier; les pigments peuvent notamment être enrobés par des composés siliconés tels que des PDMS et/ou par des polymères, notamment des polyéthylènes. On peut ainsi citer les pigments SA de Maprecos ou les pigments PI de Myoshi. En effet, on a constaté que lorsque les pigments n'étaient pas enrobés, le produit obtenu présentait un aspect non lisse et/ou cassant. Les pigments sont de préférence présents dans la phase grasse de l'émulsion.

Lorsque la composition se présente sous forme d'une solution ou d'un gel aqueux, elle peut comprendre des colorants hydrosolubles choisis parmi les colorants usuels du domaine considéré tels que le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.

Les nacres peuvent être présentes dans la composition à raison de 0-20% en poids, de préférence à un taux élevé de l'ordre de 2-15% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.

Les charges, qui peuvent être présentes dans la composition à raison de 0-20 % en poids, par rapport au poids total de la composition, de préférence 2-10%, peuvent être minérales ou de synthèse, lamellaires ou non lamellaires. On peut citer le talc, le mica, la silice, le kaolin, les poudres de Nylon et de polyéthylène, le Téflon, l'amidon, le nitrure de bore, les microsphères telles que l'Expancel (Nobel Industrie), le polytrap (Dow Corning) et les microbilles de résine de silicone (Tospearls de Toshiba, par exemple).

La composition peut comprendre en outre tout additif usuellement utilisé dans le domaine cosmétique, tel que des antioxydants, des colorants notamment hydrosolubles, des parfums, des huiles essentielles, des conservateurs, des actifs cosmétiques, des hydratants, des vitamines, des acides gras essentiels, des sphingolipides, des composés autobronzants tels que la DHA, des filtres solaires, des polymères liposolubles notamment hydrocarbonés, tels que le polybutène, les polyalkylènes, les polyacrylates et les polymères siliconés compatibles avec les corps gras.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Ces additifs peuvent être présents dans la composition à raison de 0-10% en poids.

Les compositions selon l'invention peuvent se présenter sous toutes les formes appropriées pour une application topique, notamment sous forme de sérum, de lotion, de crème, de lait, de gel aqueux, d'émulsions obtenues par dispersion d'une phase grasse dans une phase aqueuse (H/E) ou inversement (E/H), de consistance liquide ou semi-liquide, voire pâteuse ou solide.

Les émulsions selon l'invention peuvent constituer tout ou partie d'une composition cosmétique, pharmaceutique ou hygiénique.

Les compositions selon l'invention trouvent une application notamment dans le domaine du maquillage de la peau, des semi-muqueuses, des muqueuses et/ou des phanères, et se présentent alors par exemple sous la forme d'un fond de teint, d'un fard à joues ou à paupières, d'un rouge à lèvres, d'un mascara ou d'un eye-liner.

Elles peuvent également être utilisées comme base de soin pour les lèvres, ou comme produit de soin de la peau, des muqueuses, des semi-muqueuses et/ou des phanères, produit hygiénique ou pharmaceutique ou encore produit solaire ou autobronzant.

Elles trouvent également une application dans le domaine capillaire, notamment en tant que gels ou crèmes de soin des phanères telles que cheveux, cils et sourcils, ou encore en tant que gel aqueux notamment de coiffage.

L'invention est illustrée plus en détails dans les exemples suivants.

**Exemple de préparation A du polymère**

Dans un ballon de 5 litres muni d'un agitateur, d'un réfrigérant à reflux, d'un thermomètre et d'un dispositif de conduite pour l'azote et pour l'ammoniaque, on introduit 2006,2 g de tertio-butanol puis 340,0 g d'acide 2-acrylamido 2-méthylpropane sulfonique que l'on disperse dans la solution sous forte agitation. Après 30 minutes, on ajoute l'ammoniaque par le conduit supérieur du ballon et on maintient le mélange réactionnel pendant 30 minutes à température ambiante jusqu'à l'obtention d'un pH de l'ordre de 6-6,5. On introduit ensuite 32,0 g d'une solution de triméthylolpropane triacrylate à 25% dans le tertio-butanol et on chauffe jusqu'à 60°C tandis que le milieu réactionnel est simultanément rendu inerte par apport de l'azote dans le ballon. Une fois cette température atteinte, on ajoute du dilauroylperoxyde. La réaction se déclenche aussitôt, ce qui se traduit par une montée de température et par une précipitation du polymérisat. 15 minutes après le début de la polymérisation, on introduit un courant d'azote. 30 minutes après l'ajout de

l'amorceur, la température du milieu réactionnel atteint un maximum de 65-70°C. 30 minutes après avoir atteint cette température, on chauffe au reflux et on maintient dans ces conditions pendant 2 heures. On observe au cours de la réaction la formation d'une pâte épaisse. On refroidit jusqu'à la température ambiante et on filtre le produit obtenu. La pâte récupérée est ensuite séchée sous vide à 60-70°C pendant 24 heures. On obtient 391 g de poly(acide 2-acrylamido 2-méthylpropane sulfonique) réticulé et neutralisé, ayant viscosité mesurée au viscosimètre BROOKFIELD, mobile 4, à une vitesse de rotation de 100 tours/minutes dans une solution d'eau à 2 % et à 25 °C allant de 15000 cps à 35.000 cps. La viscosité du polymère sera choisie et contrôlée selon des moyens classiques en fonction de l'application cosmétique envisagée.

**Exemple de préparation B du polymère**

Dans un ballon de 5 litres muni d'un agitateur, d'un réfrigérant à reflux, d'un thermomètre et d'un dispositif de conduite pour l'azote et pour l'ammoniaque, on introduit 2006,2 g de tertio-butanol puis 340,0 g d'acide 2-acrylamido 2-méthylpropane sulfonique que l'on disperse dans la solution sous forte agitation. Après 30 minutes, on ajoute l'ammoniaque par le conduit supérieur du ballon et on maintient le mélange réactionnel pendant 30 minutes à température ambiante jusqu'à l'obtention d'un pH de l'ordre de 6-6,5. On introduit ensuite 19,2 g d'une solution de triméthylolpropane triacrylate à 25% dans le tertio-butanol et on chauffe jusqu'à 60°C tandis que le milieu réactionnel est simultanément rendu inerte par apport de l'azote dans le ballon. Une fois cette température atteinte, on ajoute du dilauroylperoxyde. La réaction se déclenche aussitôt, ce qui se traduit par une montée de température et par une précipitation du polymérisat. 15 minutes après le début de la polymérisation, on introduit un courant d'azote. 30 minutes après l'ajout de l'amorceur, la température du milieu réactionnel atteint un maximum de 65-70°C. 30 minutes après avoir atteint cette température, on chauffe au reflux et on maintient dans ces conditions pendant 2 heures. On observe au cours de la réaction la formation d'une pâte épaisse. On refroidit jusqu'à la température ambiante et on filtre le produit obtenu. La pâte récupérée est ensuite séchée sous vide à 60-70°C pendant 24 heures. On obtient 391 g de poly(acide 2-acrylamido 2-méthylpropane sulfonique) réticulé et neutralisé, ayant une viscosité mesurée au viscosimètre BROOKFIELD, mobile 4, à une vitesse de rotation de 100 tours/minutes dans une solution d'eau à 2 % et à 25 °C de l'ordre de 7000 cps.

**Exemple de composition 1**

On prépare un fond de teint ayant la composition suivante :

- oxydes de fer et de titane enrobés de PDMS 10 g
- poly(acide 2-acrylamido 2-méthylpropane sulfonique) réticulé et neutralisé par l'ammoniaque, préparé selon le procédé de l'exemple A, de viscosité à 25°C de 16000 cps en solution aqueuse à 2% 1 g
- gomme de silicone hydroxylée dans PDMS 16 g
- conservateurs qs
- eau qsp 100 g

On chauffe la phase aqueuse de manière à dissoudre les conservateurs et on y disperse le polymère; on disperse les pigments dans la phase grasse et on introduit cette phase grasse dans la phase aqueuse à température ambiante et sous agitation.
On obtient ainsi un fond de teint qui s'étale facilement sur la peau sans sensation de gras et ne transfère pas au contact d'un tissu.
Ce fond de teint confère une certaine sensation de fraîcheur lors de son application sur la peau.

**Exemple de composition 2**

On prépare un gel de maquillage ayant la composition suivante:

- oxydes de fer et de titane enrobés de PDMS 7 g
- poly(acide 2-acrylamido 2-méthylpropane sulfonique) réticulé et neutralisé par l'ammoniaque, préparé selon le procédé de l'exemple A, de viscosité à 25°C de 16000 cps en solution aqueuse à 2% 1,5 g
- mélange de gomme siliconée et de silicone volatile (polydiphényldiméthylsiloxane + cyclopentadiméthylsiloxane) 16 g
- solution aqueuse d'$\alpha$-hydroxyacides (49% de matière sèche) 1 g
- conservateurs qs
- triéthanolamine qsp pH 3,5
- eau qsp 100 g

On obtient une émulsion stable, ayant un pH de 3,5, qui permet l'obtention d'un maquillage uniforme et naturel Le produit est frais à l'application et facile à appliquer. On obtient une sensation de douceur en final. La composition présente une bonne tenue et ne transfère pas.

**Exemple 3 : Etude des propriétés de persistance des émulsions en fonction de la nature de la phase grasse**

On a réalisé différentes émulsions (fond de teint) conformes à l'invention, chaque émulsion étant différente par la nature de la phase grasse.

Les émulsions ont été préparées selon les compositions suivantes :

- phase grasse X g
- pigments enrobés PDMS 10 g
- poly(acide 2-acrylamido 2-méthylpropane sulfonique) réticulé et neutralisé par l'ammoniaque, préparé selon le procédé de l'exemple A, de viscosité à 25°C de 16000 cps en solution aqueuse à 2% 1 g
- eau qsp 100 g

On a préparé les compositions, de manière usuelle, en mélangeant les ingrédients de la phase grasse et les pigments préalablement dispersés ; on a préparé la phase aqueuse en mélangeant l'eau et le polymère puis on a chauffé à 80 °C; on a mélangé les deux phases à température ambiante sous agitation à l'aide d'une turbine.

On a ensuite déterminé les propriétés de persistance de ces émulsions.

Pour cela, on a appliqué 0,05 g de chaque émulsion sur une surface de 50 $cm^2$ sur l'avant-bras puis on a laissé sécher la composition appliquée pendant 5 minutes.

On a ensuite appliqué une bande de tissu en polyester sur la partie de l'avant-bras traité. A l'aide d'un appareil, on a animé la bande d'un mouvement de translation vertical, au contact de l'avant-bras traité. Le tissu a été maintenu tendu à l'aide d'un contrepoids créant ainsi un frottement du tissu pendant la translation. On a réalisé 10 mouvements aller et retour de frottement.

On a ensuite noté les traces colorées qui se sont éventuellement déposées sur le tissu selon la notation suivante :

tissu très taché : note = 0
tissu sans trace : note = 10

On considère qu'un fond de teint transfère peu lorsque la notation est égale ou supérieure à 8,5.

Les résultats obtenus sont reportés dans le tableau ci-après.

Il en ressort que les émulsions selon l'invention présentent de bonnes propriétés de persistance et ne transfèrent pas sur le tissu.

Ceci est également le cas lorsque des huiles d'origine végétale, ou des esters gras, sont présents dans la composition.

## TABLEAU I

| Phase grasse | Teneur (X) en gramme | Note (1 à 10) |
|---|---|---|
| Gomme de silicone hydroxylée dans PDMS (Q2-1403 de Dow Corning) | 25 | 9,5 |
| | 16 | 9,5 |
| Gomme polydiphényldiméthylsiloxane dans cycloD5 (15/85) | 25 | 9,5 |
| Polydiméthylsiloxane (viscosité 10 cSt) | 12 | 10 |
| Huile de silicone phénylée (DC556 de Dow Corning) | 16 | 9,5 |
| Huile d'amande d'abricot | 25 | 8,5 |
| Neopentanoate d'isostéaryle | 12 | 10 |
| Mélange Q2-1403 + huile d'abricot | 8 + 8 | 9 |
| Mélange Q2-1403 + neopentanoate d'isostéaryle | 8 + 8 | 9 |
| | 5 + 11 | 9,5 |
| | 14 + 11 | 8,5 |
| Mélange PDMS 10 cSt + Q2-1403 | 12,5 + 12,5 | 9 |

## Revendications

1. Utilisation d'un polymère poly(acide 2-acrylamido 2-méthylpropane sulfonique) réticulé et neutralisé à au moins 90% dans une composition cosmétique, dermatologique, hygiénique et/ou pharmaceutique, afin de limiter, diminuer et/ou de supprimer le transfert et/ou la migration de ladite composition.

2. Utilisation d'un polymère poly(acide 2-acrylamido 2-méthylpropane sulfonique) réticulé et neutralisé à au moins 90%, dans une composition cosmétique, dermatologique, hygiénique et/ou pharmaceutique, afin d'améliorer la tenue de ladite composition.

3. Utilisation d'un polymère poly(acide 2-acrylamido 2-méthylpropane sulfonique) réticulé et neutralisé à au moins 90% dans une composition cosmétique, dermatologique, hygiénique et/ou pharmaceutique, comme agent permettant de limiter, diminuer et/ou de supprimer le transfert et/ou la migration de ladite composition, et/ou comme agent permettant d'améliorer la tenue de ladite composition.

4. Utilisation selon l'une des revendications précédentes, dans laquelle le polymère comprend, distribués de façon aléatoire :

   a) de 90 à 99,9% en poids de motifs de formule générale (1) suivante :

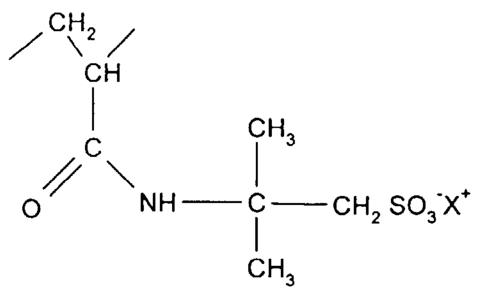

(1)

   dans laquelle $X^+$ désigne un cation ou un mélange de cations, au plus 10% molaire des cations $X^+$ pouvant être des protons $H^+$ ; et

   b) de 0,01 à 10% en poids de motifs réticulants provenant d'au moins un monomère ayant au moins deux doubles liaisons oléfiniques ; les proportions en poids étant définies par rapport au poids total du polymère.

5. Utilisation selon la revendication 4, dans laquelle le polymère comprend de 98 à 99,5 % en poids de motifs de formule (1) et de 0,2 à 2 % en poids de motifs réticulants.

6. Utilisation selon l'une des revendications 4 à 5, dans laquelle le cation $X^+$ représente un cation ou un mélange de cations choisis parmi un proton, un cation de métal alcalin, un cation équivalent de celui d'un métal alcalino-terreux ou l'ion ammonium.

7. Utilisation selon l'une des revendications 4 à 6, dans laquelle les monomères de réticulation ayant au moins deux doubles liaisons oléfiniques sont choisis parmi le dipropylèneglycol-diallyléther, les polyglycol-diallyléthers, le triéthylèneglycoldivinyléther, l'hydroquinone-diallyl-éther, le tétrallyl-oxéthanoyle ou d'autres allyl ou vinyléthers alcools polyfonctionnels, le diacrylate de tétraéthylèneglycol, la triallylamine, le triméthylolpropane-diallyléther, le méthylen-bis-acrylamide et/ou le divinylbenzène.

8. Utilisation selon l'une des revendications 4 à 6, dans laquelle les monomères de réticulation ayant au moins deux doubles liaisons oléfiniques sont choisis parmi ceux répondant à la formule générale (2) suivante :

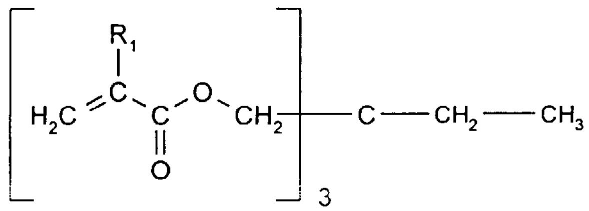

(2)

dans laquelle $R_1$ désigne un atome d'hydrogène ou un radical alkyle en $C_1$-$C_4$.

**9.** Utilisation selon l'une des revendications précédentes, dans laquelle le polymère présente une viscosité mesurée au viscosimètre BROOKFIELD, mobile 4, à une vitesse de rotation de 100 tours/minutes, à 25°C, et en solution aqueuse à 2 % en poids, supérieure ou égale à 1000 cps et plus préférentiellement allant de 5000 cps à 40000 cps et plus particulièrement de 6500 cps à 35000 cps.

**10.** Utilisation selon l'une des revendications précédentes, dans laquelle le polymère est présent à une concentration de 0,01 à 20% en poids par rapport au poids total de la composition, de préférence 0,1 à 5% en poids, et plus préférentiellement 0,4 à 2% en poids.

**11.** Utilisation selon l'une des revendications précédentes, dans laquelle la composition comprend en outre au moins un composé acide choisi parmi les hydroxyacides et les α- et β-cétoacides.

**12.** Utilisation selon l'une des revendications précédentes, dans laquelle la composition se présente sous la forme d'une émulsion huile-dans-eau, d'une émulsion eau-dans-huile, d'une émulsion multiple, d'un gel aqueux, d'une solution aqueuse, hydroalcoolique ou multiphasée, notamment eau/poudre et eau/huile/poudre.

**13.** Utilisation selon l'une des revendications précédentes, dans laquelle la composition se présente sous la forme d'un produit de maquillage de la peau, des semi-muqueuses, des muqueuses et/ou des phanères, tel qu'un fond de teint, un fard à joues ou à paupières, un rouge à lèvres, un mascara ou un eye-liner; d'une base de soin pour les lèvres; d'un produit de soin de la peau, des muqueuses, des semi-muqueuses et/ou des phanères; d'un produit hygiénique ou pharmaceutique; d'un produit solaire ou autobronzant; d'un produit capillaire.

**14.** Composition cosmétique, dermatologique, hygiénique et/ou pharmaceutique, se présentant sous la forme de, et/ou comprenant, une émulsion huile-dans-eau, une émulsion eau-dans-huile, une émulsion multiple ou une solution multiphasée, comprenant en outre au moins un polymère poly(acide 2-acrylamido 2-méthylpropane sulfonique) réticulé et neutralisé à au moins 90%, et des pigments.

**15.** Composition cosmétique, dermatologique, hygiénique et/ou pharmaceutique, se présentant sous la forme de, et/ou comprenant, un gel aqueux, une solution aqueuse, hydroalcoolique ou multiphasée, comprenant en outre au moins un polymère poly(acide 2-acrylamido 2-méthylpropane sulfonique) réticulé et neutralisé à au moins 90%, et des colorants hydrosolubles.

**16.** Composition selon l'une des revendications 14 à 15, dans laquelle le polymère comprend, distribués de façon aléatoire :

a) de 90 à 99,9% en poids de motifs de formule générale (1) suivante :

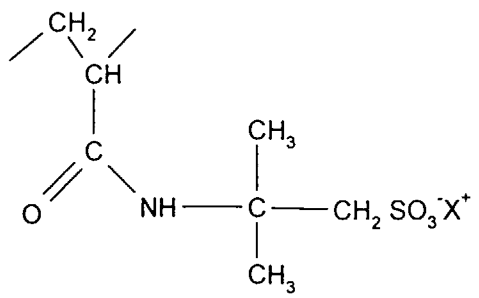

(1)

dans laquelle $X^+$ désigne un cation ou un mélange de cations, au plus 10% molaire des cations $X^+$ pouvant être des protons $H^+$ ; et

b) de 0,01 à 10% en poids de motifs réticulants provenant d'au moins un monomère ayant au moins deux doubles liaisons oléfiniques ; les proportions en poids étant définies par rapport au poids total du polymère.

**17.** Composition selon la revendication 16, dans laquelle le polymère comprend de 98 à 99,5 % en poids de motifs de formule (1) et de 0,2 à 2 % en poids de motifs réticulants.

**18.** Composition selon l'une des revendications 16 à 17, dans laquelle le cation $X^+$ représente un cation ou un mélange de cations choisis parmi un proton, un cation de métal alcalin, un cation équivalent de celui d'un métal alcalino-terreux ou l'ion ammonium.

**19.** Composition selon l'une des revendications 16 à 18, dans laquelle les monomères de réticulation ayant au moins deux doubles liaisons oléfiniques sont choisis parmi le dipropylèneglycol-diallyléther, les polyglycol-diallyléthers, le triéthylèneglycol-divinyléther, l'hydroquinone-diallyl-éther, le tétrallyl-oxéthanoyle ou d'autres allyl ou vinyléthers alcools polyfonctionnels, le diacrylate de tétraéthylèneglycol, la triallylamine, le triméthylolpropane-diallyléther, le méthylen-bis-acrylamide et/ou le divinylbenzène.

**20.** Composition selon l'une des revendications 16 à 18, dans laquelle les monomères de réticulation ayant au moins deux doubles liaisons oléfiniques sont choisis parmi ceux répondant à la formule générale (2) suivante :

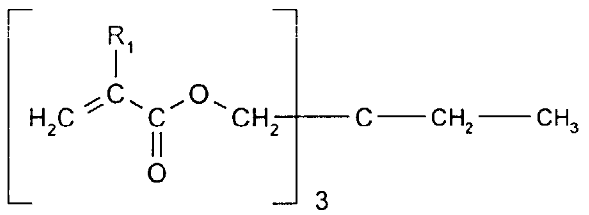

(2)

dans laquelle $R_1$ désigne un atome d'hydrogène ou un radical alkyle en $C_1$-$C_4$.

**21.** Composition selon l'une des revendications 14 à 20, dans laquelle le polymère présente une viscosité mesurée au viscosimètre BROOKFIELD, mobile 4, à une vitesse de rotation de 100 tours/minutes, à 25°C, et en solution aqueuse à 2 % en poids, supérieure ou égale à 1000 cps et plus préférentiellement allant de 5000 cps à 40000 cps et plus particulièrement de 6500 cps à 35000 cps.

**22.** Composition selon l'une des revendications 14 à 21, dans laquelle le polymère est présent à une concentration de 0,01 à 20% en poids par rapport au poids total de la composition, de préférence 0,1 à 5% en poids, et plus préférentiellement 0,4 à 2% en poids.

**23.** Composition selon l'une des revendications 14 à 22, dans laquelle la phase aqueuse représente 15 à 99,5 % en poids par rapport au poids total de la composition.

**24.** Composition selon l'une des revendications 14 à 23, comprenant en outre un monoalcool inférieur en $C_2$-$C_6$ et/ou

un polyol tel que le glycérol, le butylèneglycol, l'isoprène glycol, le propylèneglycol, le polyéthylèneglycol.

**25.** Composition selon la revendication 14, comprenant en outre au moins une huile d'origine animale, végétale, minérale ou synthétique, et/ou au moins une gomme de silicone.

**26.** Composition selon la revendication 25, dans laquelle l'huile est choisie parmi les huiles siliconées volatiles; les huiles volatiles hydrocarbonées; les huiles non volatiles siliconées, éventuellement phénylées; l'huile de paraffine, de vaseline, le perhydrosqualène, l'huile d'abricot, l'huile de germes de blé, d'amande douce, de calophyllum, de sésame, de macadamia, de pépins de raisin, de colza, de coprah, d'arachide, de palme, de ricin, d'avocat, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides, les huiles fluorées et perfluorées.

**27.** Composition selon la revendication 25, dans laquelle la gomme de silicone est choisie parmi les gommes phénylées et/ou hydroxylées.

**28.** Composition selon la revendication 14, se présentant sous la forme d'une émulsion dont la phase grasse ne comprend que des corps gras siliconés.

**29.** Composition selon l'une des revendications 14 à 28, comprenant en outre au moins un composé acide.

**30.** Composition selon la revendication 29, dans laquelle le composé acide est choisi parmi les hydroxyacides et les $\alpha$- et $\beta$-cétoacides.

**31.** Composition selon la revendication 30, dans laquelle l'hydroxyacide est un $\alpha$-hydroxyacide.

**32.** Composition selon l'une des revendications 29 à 31, dans laquelle le composé acide est présent à une teneur de 0 à 10 % en poids, par rapport au poids total de la composition, de préférence de 0,2 % à 5 % en poids.

**33.** Composition selon la revendication 14, comprenant 2-15% en poids, par rapport au poids total de la composition, de pigments et/ou nanopigments.

**34.** Composition selon la revendication 14, dans laquelle les pigments et/ou nanopigments sont choisis parmi les dioxydes de titane, de zirconium ou de cérium, les oxydes de zinc, de fer ou de chrome, les nanotitanes, le bleu ferrique, le noir de carbone, les laques de baryum, strontium, calcium, aluminium.

**35.** Composition selon l'une des revendications 14 ou 33 à 34, se présentant sous la forme d'une émulsion huile-dans-eau et dans laquelle les pigments sont hydrophobes ou rendus hydrophobes.

**36.** Composition selon la revendication 35, dans laquelle les pigments sont enrobés par des composés siliconés tels que des PDMS et/ou par des polymères, notamment des polyéthylènes.

**37.** Composition selon la revendication 15, dans laquelle les colorants hydrosolubles sont choisis parmi le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.

**38.** Composition selon l'une des revendications 14 à 37, se présentant sous la forme d'un produit de maquillage de la peau, des semi-muqueuses, des muqueuses et/ou des phanères, tel qu'un fond de teint, un fard à joues ou à paupières, un rouge à lèvres, un mascara, un eye-liner; d'une base de soin pour les lèvres; d'un produit de soin de la peau, des muqueuses, des semi-muqueuses et/ou des phanères; d'un produit hygiénique ou pharmaceutique; d'un produit solaire ou autobronzant; d'un produit capillaire.

**39.** Composition de maquillage ou de soin sans transfert, comprenant au moins un polymère poly(acide 2-acrylamido 2-méthylpropane sulfonique) réticulé et neutralisé à au moins 90%.

**40.** Procédé pour limiter, diminuer et/ou empêcher le transfert d'une composition cosmétique, dermatologique, hygiénique et/ou pharmaceutique, notamment une composition de maquillage ou de soin de la peau, des muqueuses, des semi-muqueuses et/ou des phanères, consistant à introduire dans ladite composition au moins un polymère

poly(acide 2-acrylamido 2-méthylpropane sulfonique) réticulé et neutralisé à au moins 90%.

Office européen des brevets

# RAPPORT DE RECHERCHE EUROPEENNE

Numero de la demande
EP 97 40 1205

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | US 5 114 706 A (DUVEL LANE A) 19 mai 1992 | 1-3,6, 10,12, 14,39,40 | A61K7/48 A61K7/00 A61K7/02 A61K7/06 |
| | * colonne 3, ligne 1-12 * * colonne 7, ligne 67-68 * | | |
| Y | * colonne 8, ligne 1-29 * * colonne 10, ligne 9-19 * * colonne 10, ligne 45-49 * * exemples 1-21 * --- | 1-40 | |
| X | US 4 695 453 A (TUOMINEN FRANCIS W ET AL) 22 septembre 1987 * le document en entier * --- | 1-3, 10-13, 39,40 | |
| X | EP 0 152 095 A (HENKEL CORP) 21 août 1985 * page 11; revendications 1-5,7,8,11 * --- | 1-4,6, 10, 12-14, 16,18, 22,38-40 | |
| | | | **DOMAINES TECHNIQUES RECHERCHES (Int.Cl.6)** |
| Y | FR 2 528 699 A (OREAL) 23 décembre 1983 * le document en entier * --- | 1-40 | A61K |
| A | GB 1 110 240 A (H. W. HEINRICH) 27 juillet 1967 * le document en entier * ----- | 1-40 | |

Le présent rapport a été établi pour toutes les revendications

| Lieu de la recherche | Date d'achèvement de la recherche | Examinateur |
|---|---|---|
| LA HAYE | 30 septembre 1997 | Sierra Gonzalez, M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)